# EUROPEAN PATENT SPECIFICATION

(11) **EP 0 648 836 B1**
(45) Date of publication and mention of the grant of the patent: **09.01.2002**
(21) Application number: 94113378.7
(22) Date of filing: 26.08.1994
(51) Int. Cl.: C12N 15/12, C07K 14/00, C07K 14/47, C12N 5/10, G01N 33/68, C12Q 1/68, A61K 38/17, A61K 31/70, C12N 9/00

(54) **MHC class II transactivator (CIITA) and uses thereof**
MHC-Klasse-II Transaktivator (CIITA) und dessen Anwendungen
Transactivateur du complexe majeur d'histomcompatibilité de la classe II et ses utilisations

(30) Priority: 26.08.1993 EP 93113665
(43) Date of publication of application: 19.04.1995
(73) Proprietor: NovImmune SA, 1207 Genève (CH)
(72) Inventor: Mach, Bernard, Prof., 1292 Chambésy Genève (CH)
(74) Representative: VOSSIUS & PARTNER

(56) References cited:
- CELL, vol.75, 8 October 1993, CAMBRIDGE, NA US pages 135 - 146 STEIMLE, V. ET AL.; 'Complementation cloning of an MHC Class II transactivator mutated in hereditary MHC class II deficiency (or Bare Lymphocyte Syndrome)'
- EXPERIENTIA, vol.49, 1993, BASEL,CH page A7 STEIMMLE, V. ET AL.; 'Cloning of an HLA class II transactivator defective in a form of primary immunodeficiency by genetic complementation'

## Description

The invention relates to transacting proteins which are essential for the general control of vertebrate MHC class II gene expression, to DNA sequences encoding same and to substances which are capable of inhibiting the synthesis of said proteins. Furthermore, the invention relates to corresponding recombinant DNA molecules, transformed microorganisms and their use for the production of said proteins. The invention additionally relates to pharmaceutical compositions for the treatment of diseases which are associated with an impaired or aberrant expression of MHC class II genes.

Class II major histocompatibility (MHC) antigens are heterodimeric transmembrane glycoproteins. Their expression at the surface of antigen-presenting cells is essential for the recognition of foreign antigens by the T cell receptor. T cell activation and antigen presentation depend on the level of expression of class II antigens on individual cells. Regulation of expression of class II genes is therefore an important aspect of the control of both normal and abnormal immune responses.

In humans, the genes encoding the a and b chains of the HLA-DP, HLA-DQ and HLA-DR class II molecules are clustered in the D region of the MHC on chromosome 6. These genes are subjected to tight and complex regulatory controls. Their expression is generally coordinated, and restricted primarily to cells of the immune system such as B lymphocytes, activated T lymphocytes, macrophages, dendritic cells, and certain specialized cells such as Kupffer cells and Langerhans cells. In certain class II negative cells, expression can be induced by stimulation with lymphokines such as interferon c or interleukin 4.

MHC class II genes represent a particularly complex case of regulated gene expression. Since presentation of foreign antigens to helper T cells as well as thymic education of T lymphocytes require normal expression of MHC class II molecules, the regulation of these genes is essential for a normally functioning immune system. Genes encoding MHC class II antigens were indeed initially described as "immune response genes" [Benaceraff, Science 212 (1981), 1229-1238]. This regulation concerns not only the level of expression of class II molecules but also the very restricted cell type specificity, since most cells in the body are normally MHC class II negative. The complexity of this regulation involves two distinct modes of control, namely constitutive expression in cells such as B lymphocytes and inducible expression in certain cell types, such as monocytes or fibroblasts. Finally, this family of genes encodes the a and b chains of three different HLA class II isotypes and is generally regulated in a global manner.

A number of protein factors have been shown to be capable of binding in vitro to functionally essential sequence motifs in MHC class II promoters, in particular to the conserved X and Y boxes [Glimcher et al., Ann. Rev. Immunol. 10 (1992), 13-49]. In addition, the state of occupancy of class II promoters has been analyzed in vivo [Kara et al., Science 252 (1991), 709-712]. In spite of this progress, the basic mechanisms that control MHC class II gene regulation are still elusive and no factor has been shown to function directly as a transcription activator of class II genes.

Several autoimmune diseases, such as insulin-dependent diabetes, multiple sclerosis, rheumatoid arthritis and lupus erythematosis are thought to be due, at least in part, to aberrant expression of HLA class II antigens on cells that normally should not express them. Abnormal T cell activation then leads to the autoimmune process. Treatment of animals suffering from autoimmune diseases with antibodies directed against MHC class II molecules have already given encouraging results.

Furthermore, it has been shown that patients suffering from congenital severe combined immunodeficiency (SCID) show an absence of MHC class II gene expression, which is caused by the absence of a factor binding to the X-Box of a promoter.

It would consequently be very desirable to be able to down-regulate the expression of MHC class II genes in order to prevent or treat autoimmune diseases or to restore, induce or boost the expression of MHC class II genes in the case of diseases which are caused by missing or impaired expression of MHC class II genes, e.g. in gene therapy for class II deficient immunodeficiencies. Additional examples for the use of proteins essential for the expression of MHC class II genes include vaccination to improve the immunogenicity and to make the relevant cells better APCs (antigen-presenting cells) (for instance, by incorporating the CIITA gene in any form of live vaccine, such as vaccinia virus-based vaccines), or gene therapy for cancer treatment, inducing the cancer cells to express MHC class II molecules and thus to behave as better APCs. Thus, access to proteins possessing the capability of modulating the regulations of MHC class II gene expression or restoring, inducing or boosting the expression of said genes if impaired could provide ways for the treatment of the disorders mentioned above.

Therefore, the technical problem underlying the present invention is to isolate and identify proteins essential for the expression of MHC class II genes and to provide them in purified form. Furthermore, the technical problem underlying the present invention is to provide proteins with the biological properties of a class II transactivator, DNA sequences encoding said class II transactivator and/or related proteins, recombinant DNA molecules containing these DNA sequences and microorganisms transformed with said recombinant DNA molecules. Still further, the technical problem is to provide recombinant DNA processes for the production of said proteins in which the transformed microorganisms are used. In addition, it is the technical problem to provide pharmaceutical compositions containing said proteins or corresponding DNA sequences.

The above technical problem is solved by providing the embodiments of the present invention which are characterized in the claims.

Accordingly, purified proteins are provided which normally occur in vertebrate cells expressing MHC class II genes, and which are involved in the general control of the expression of vertebrate MHC class II genes and DNA sequences encoding the proteins of the present invention.

Such proteins could be obtained by the following approach:

A regulatory mutant B cell line that exhibits no detectable defect in protein binding to HLA class II promoters was used for the cloning and identification of the MHC class II regulatory gene affected, using complementation of the mutant phenotype by cDNAs derived from an MHC class II positive B cell. Selection was based not only on re-expression of endogenous class II genes but also on the re-activation of an MHC class II promoter (inactive in the mutant cells) driving a gene for antibiotic resistance. Cloning sites unable to rejoin upon ligation were created to ensure the efficiency of a large cDNA insertion. Carefully size-selected cDNA was used and the cDNA libraries that led to the successful selection of the 4.5 kb CIITA cDNA contained inserts of average sizes of more than 3.5 kb. In addition, the transfection conditions for B lymphocytes and the immuno-selection procedure for surface expression with magnetic beads were thoroughly optimized.

Cloning of CIITA cDNA by complementation of the commercially available class II negative B cell line RJ2.25 has enabled the following conclusions to be drawn: CIITA is indeed the gene affected in this mutant, and deletions in the two CIITA alleles explain the absence of intact CIITA mRNA. One allele has only an internal deletion in the CIITA gene and it appears that transcriptional readthrough results in a truncated transcript. Restoration of MHC class II gene expression by CIITA cDNA establishes a functional role for this factor in the control of constitutive MHC class II expression in B lymphocytes. CIITA behaves as a transactivator of all three HLA class II isotypes, DR, DQ and DP, and there is no evidence of locus-specific control by this factor.

CIITA is a novel protein of 1130 amino acids whose mRNA is expressed at a low level. Its structure is not very informative concerning its mode of action. The NH₂ terminal portion of the molecule has some of the attributes of certain transcription activation domains [Mitchell et al., Science 245 (1989), 371-378], in particular a region of acidic amino acids followed by three short stretches rich in proline, threonine and serine residues. Certain transcription factors have similar activation domains. A sequence corresponding to an ATP/GTP binding site was recognized around amino acids 420 to 427 and mutagenesis at those positions will be informative. Finally, there is a leucine-rich region around amino acids 979 to 1061 that shows a weak homology with the NH₂ terminal portion of an RNA binding protein of yeast [Traglia et al., Mol. Cell. Biol. 9 (1989), 2989-2999]. Since this weak homology concerns primarily leucine residues within a leucine-rich region, it may not be indicative of evolutionary relatedness. In the absence of an obvious DNA binding domain in the CIITA sequence, one can speculate that it may function as an adapter between factors known to bind to the X and Y boxes of MHC class II promoters and the transcription machinery.

Furthermore, the inventors have found that the transactivator CIITA not only regulates the constitutive expression of MHC class II genes in cells such as B lymphocytes, it also controls the inducible expression of these same genes in other cell types, for instance induction by interferon gamma or by TNF. It is therefore a factor involved in the general control of MHC class II gene expression. Its structure is not related to other factors.

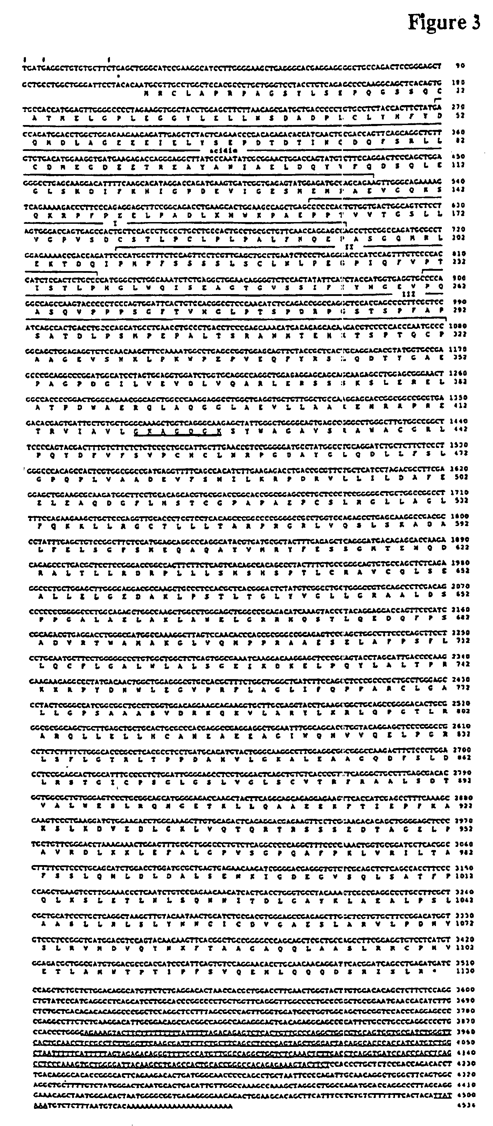

In one embodiment, the present invention relates to DNA sequences encoding the proteins of the present invention. Such DNA sequences can for instance be isolated by expression cloning by genetic complementation of MHC class II regulatory mutants as described above and following the basic procedure given in the examples.
A preferred DNA sequence of the present invention which encodes the entire class II transactivator (CIITA) is given in Figure 3. It will be understood that the person skilled in the art provided with the sequence shown in Figure 3 would be in a position to use this DNA sequence, a fragment thereof, or a corresponding oligonucleotide in order to isolate related DNA sequences from genomic or cDNA libraries of vertebrates such as mammals, or from other human individuals. Related DNA sequences are for instance allelic human DNA sequences.
On the other hand, the person skilled in the art, provided with the screening strategy of the present invention, would be in a position to isolate corresponding DNA sequences from other vertebrates, such as mammals, or from other human individuals.
Furthermore, the DNA sequences given in Figure 3 or DNA sequences which are related to them can be obtained by chemical synthesis.
All the above DNA sequences are within the scope of the present invention. Thus, the present invention also relates to DNA sequences which hybridize to the DNA sequence of Figure 3 under conventional hybridization conditions.

The present invention furthermore relates to DNA sequences which are related to the above DNA sequences by the degeneration of the genetic code and to DNA sequences encoding a protein with the amino acid sequence shown in Figure 3.

Said DNA sequences, encoding the active CIITA transactivator protein, are useful to induce or boost the expression of MHC class II molecules in situations such as vaccination, to improve the immunogenicity of the relevant cells, to boost peptide-specific immunogenicity, cancer treatment by gene therapy, to improve the immunogenicity of the cancer cells, and in gene therapy for MHC class II deficiency disease.

In a further embodiment, the present invention relates to nucleotide sequences, i.e. to RNA or DNA sequences, which are complementary to the coding strand of the above-mentioned DNA sequences. These complementary nucleotide sequences, also referred to as "antisense" RNA, DNA, or ribozymes, are known to be capable of inhibiting the synthesis of the protein encoded by the relevant gene. Said complementary sequences are useful for repression of MHC class II gene expression, e.g. in the case of autoimmune diseases due to aberrant T cell activation, organ transplantation, especially bone marrow transplantation, and to generate MHC class II-negative animals as xenogenic organs donors.
The person skilled in the art provided with the DNA sequences mentioned above will be in a position to produce and utilize the corresponding antisense RNA, DNA, or ribozymes.
Ribozymes which are composed of a single RNA chain are RNA enzymes, i.e. catalytic RNAs, which can intermolecularly cleave a target RNA, for example the mRNA transcribed from the CIITA gene. It is now possible to construct ribozymes which are able to cleave the target RNA at a specific site by following the strategies described in the literature [Tanner et al. in Antisense Research and Applications, CRC Press, Inc. (1993), 415-426]. The two main requirements for such ribozymes are the catalytic domain and regions which are complementary to the target RNA and which allow them to bind to its substrate, which is a prerequisite for cleavage.

In still a further embodiment, the present invention relates to CIITA inhibitors which fulfill the same purpose as the latter RNA or DNA sequences or ribozymes mentioned above, i.e. repression of MHC class II gene expression. Such inhibitors can be, for instance, structural analogues of the CIITA protein that act as antagonists. In addition, such inhibitors comprise molecules identified by the use of recombinant CIITA.

Furthermore, the present invention relates to recombinant vectors containing the DNA and nucleotide sequences of the present invention.

A preferred recombinant vector is a plasmid in which the inserted gene is in operable linkage with an expression control sequence.

Another preferred recombinant vector is pDVP10-1, containing a 4543 bp cDNA-insert in plasmid vector DV which encodes the human MHC class II transactivator. This cDNA was synthesized from mRNA of the HLA class II positive parental B cell line Raji.
This plasmid was deposited on July 19, 1993 as a strain of Escherichia coli (E. coli pDVP10-1) containing the plasmid. The accession number is DMS 8422.

The invention also relates to host organisms which are transformed with the recombinant DNA molecules of the present invention. Such host organisms are preferably bacteria such as E. coli or Bacillus subtilis, yeast such as species or strains of Saccharomyces (e.g. Saccharomyces cerevisiae), and other eukaryotic host cells such as insect or mammalian cells.

Still further, the present invention relates to a method for the identification and isolation of MHC class II transactivators comprising the following steps:
(a) construction of a cDNA expression library from the mRNA of Raji cells;
(b) transforming an MHC class II mutant with the DNA of said library;
(c) screening the transformants by genetic complementation using re-expression of the endogenous MHC class II gene as a selection marker; and
(d) isolating and sequencing plasmids which show complementation.

Purified proteins displaying CIITA activity and being essential for the general control of expression of MHC class II genes in vertebrates are a further embodiment of the present invention.

The term "proteins essential for the general control of expression of MHC class II genes" means transacting proteins that can interact with control regions of MHC class II genes and which are essential for the expression of said genes.

A further preferred embodiment is a protein having the above-mentioned properties which naturally occurs in mammalian, preferably human, cells expressing MHC class II genes.

The invention further relates to the proteins mentioned above which are recombinantly produced using the recombinant vectors and host cells of the invention. Such proteins can be used to screen for and identify CIITA inhibitors, for example by exploiting the capability of potential inhibitors to bind to said proteins under appropriate conditions. such CIITA inhibitors can also be designed on the basis of the threedimensional structure of CIITA, an information that can be obtained from recombinant CIITA using state of the art technology.

Furthermore, the present invention relates to processes for the production of proteins of the present invention comprising the steps of cultivating a transformed host organism of the present invention under suitable conditions in a culture medium and recovering the resulting expression product from the medium. Optionally, the recovered expression product can be purified according to conventional methods, such as chromatographies using ion exchange resins or affinity chromatography material, e.g. monoclonal or polyclonal antibodies attached to a matrix.

The present invention also relates to pharmaceutical compositions which contain at least one of the proteins of the present invention or the "antisense" RNA or DNA, ribozymes or inhibitors. These pharmaceutical compositions optionally also contain pharmaceutically acceptable carriers and/or additives.
The dosage depends on the condition of the patient and the symptoms of the disease.

In particular, the pharmaceutical compositions of the invention containing the proteins or the DNA of the present invention can be used for the treatment of diseases wherein an increase of the expression of MHC class II genes is desirable. Immunodeficiency due to a defect in the MHC class II transactivator corresponds to such a clinical condition. The mechanism of the beneficial effect will be the replacement of a defective MHC class II transactivator gene or protein in the patients.

The pharmaceutical compositions of the invention containing the proteins or the DNA of the present invention can also be used in medical situations where an increased expression of MHC class II molecules is desirable, such as for gene therapy in cancer cells or in the composition of vaccines, to render the relevant cells more immunogenic or to boost peptide-specific immunogenicity. In these cases, the introduction of the CIITA gene (DNA) into live cells can be done by a number of different procedures, involving different vectors.

Furthermore, the pharmaceutical compositions of the present invention which contain the above-mentioned nucleotide sequences or inhibitors can be used for the treatment of diseases wherein a decrease of the level of the expression of MHC class II genes is desirable or for the generation of MHC class II negative transgenic animals as a source of organs for xenogenic transplantation or of cells for universal cell transplants. Among other things, these include autoimmune diseases wherein an aberrant and excessive expression of HLA class II molecules at the surface of certain cells is thought to be responsible for the autoimmune pathological processes. These include insulin dependent diabetes (IDD), multiple sclerosis (MS), lupus erythematosis (LE) and rheumatoid arthritis (RA). The aberrant expression of MHC class II genes and proteins has been documented in certain animal models of these diseases. Furthermore, animal models of some of these autoimmune diseases have been treated successfully with antibodies directed against MHC class II molecules, pointing to the desirability of down-regulation of the expression of MHC class II genes in autoimmune diseases.

### Brief Description of the Drawings

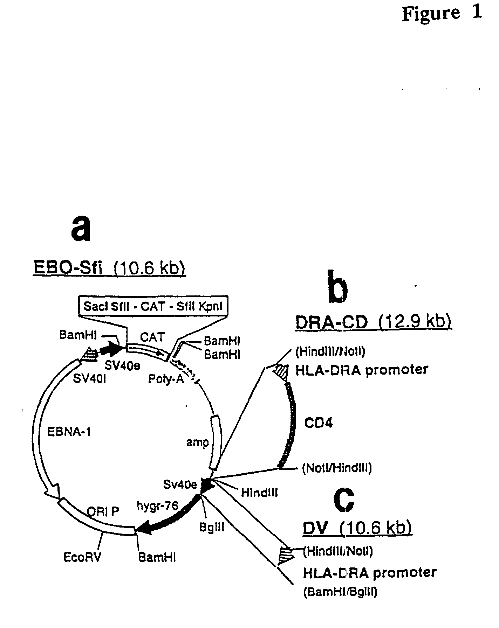

### Figure 1. Restriction map of the EBO-Sfi and derived cDNA expression vectors DRA-CD and DV.

(a) Vector EBO-Sfi contains the hygromycin resistance gene (hygr-76) from plasmid pTG76 [Giordano et al., Gene 88 (1990), 285-288]. The cDNA cloning cassette consists of two inverted SfiI sites separated by a bacterial chloramphenicol acetyl transferase gene (SfiI-CAT-gene-SfiI). Cloned cDNAs are driven by the simian virus 40 early (SV40e) promoter and polyadenylation signal (Poly-A). Open boxes indicate the EBV origin of replication (ORI P), the EBV nuclear antigen gene 1 (EBNA-1) under the control of the simian virus 40 late promoter (SV401) and the bacterial ampicillin resistance gene (amp).
(b) Vector DRA-CD was generated from EBO-Sfi by insertion of a CD4 cDNA [Maddon et al., Cell 42 (1985), 93-104] under control of the 300 bp HLA DRA promoter and a 130 bp polyadenylation signal into the unique HindIII site of EBO-Sfi.
(c) For generation of vector DV the SV40 early promoter driving the hygr-76 gene was replaced by the 300 bp HLA DRA promoter via the unique HindIII and BgIII sites.

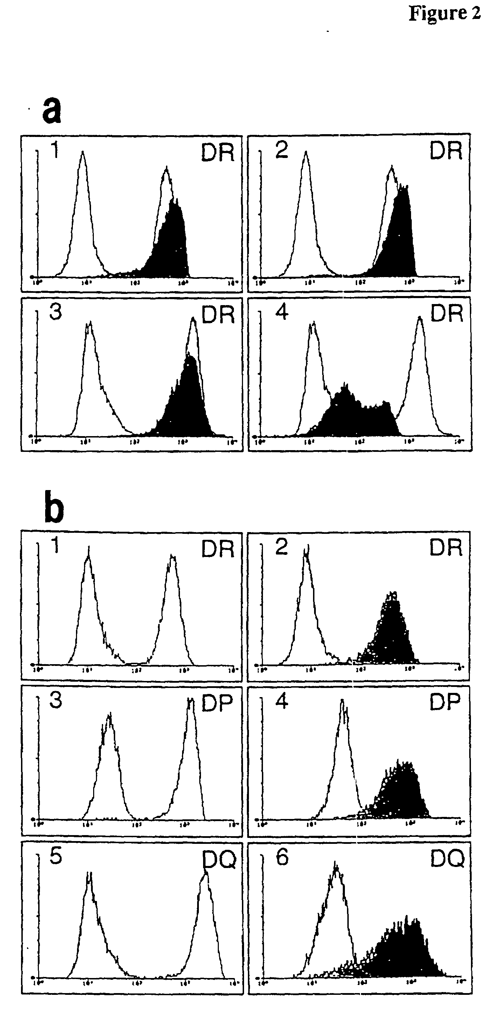

### Figure 2. Isolation of CIITA cDNA by expression cloning and correction of HLA class II expression in RJ2.25 cells.

(a) Selection of HLA-DR positive cells. RJ2.25 cells transfected with plasmid DNA from DV-pools DVP6, -7 and -10 (panels 1 to 3) and one DRA-CD pool (DRA-CDP1, panel 4) were selected for Hygromycin B resistance and sorted twice (DV transfectants) or three times (DRA-CD transfectants) with the HLA DR specific antibody 2.06 and magnetic beads. The sorted populations were stained with the HLA DR antibody L243 and analyzed by FACS (shaded profiles). As negative and positive controls, RJ2.25 and Raji cells were analyzed in the same way (open profiles).
(b) CIITA cDNA restores expression of all three HLA class II isotypes. RJ2.25 and Raji cells (panels 1, 3, 5) or RJ2.25 cell transfected with DRA-CD and DRA-CD/CIITA (open and shaded profiles in panels 2, 4, 6) were stained with antibodies specific for HLA DR (L243; panels 1, 2), -DP (B7/21; panels 3, 4) and -DQ (Tü22; panels 5, 6) and analyzed by FACS (Fluorescence Activated Cell Sorter).

### Figure 3. Nucleotide and predicted amino acid sequence of the CIITA cDNA.

The complete nucleotide sequence of cDNA clone pDVP10-1 and the deduced amino acid sequence of CIITA are shown. The 5' ends of three independent clones are indicated by "#", the upstream in-frame stop codon and the stop-codon at nt position 3506 are indicated by "*". The N-terminal regions rich in glutamate/aspartate (marked "acidic") and the stretches rich in proline/serine/threonine (marked "I, II, III") are overlined. The ATP/GTP binding cassette is double underlined. The Alu-repeat and the polyadenylation signal in the 3' untranslated region are underlined. Numerous potential protein kinase C and casein kinase II sites (13 and 15) are present in the sequence but not indicated in the figure.

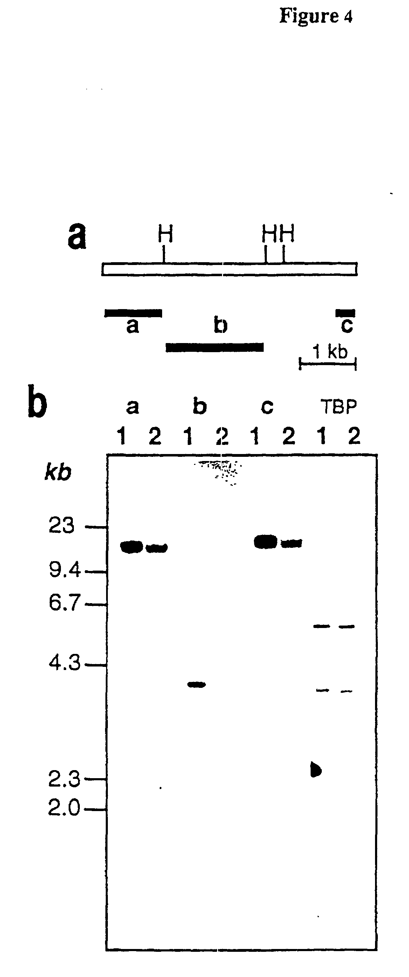

### Figure 4. Evidence for deletion in the CIITA gene in mutant cell line RJ2.25.

(a) Schematic representation of the CIITA cDNA with its three internal HindIII sites (H). The three fragments of the CIITA cDNA used as Southern hybridization probes are indicated by filled bars.
(b) 10 µg of genomic DNA were digested with HindIII, fractionated in a 0.7% agarose gel, denatured, transferred and hybridized to CIITA- or TBP-specific probes. Two identical filters were prepared with DNA from Raji (lane 1) and RJ2.25 (lane 2). The first filter was hybridized to a 1.1 kb HindIII probe from the 5' end of CIITA (probe A). After stripping, the same filter was rehybridized to probe B, the central 1.8 kb HindIII fragment of CIITA cDNA. The second filter was first hybridized to probe C spanning nucleotides 4152 to 4520 of CIITA. To confirm equal quantities of DNA in both lanes this filter was then stripped and rehybridized to a probe from the 3' end of human TBP cDNA [Kao et al., Science 248 (1990), 1646-1650]. The CIITA specific bands show lengths of 13 kb, 3.8 kb and 14 kb for probes A, B and C, respectively.

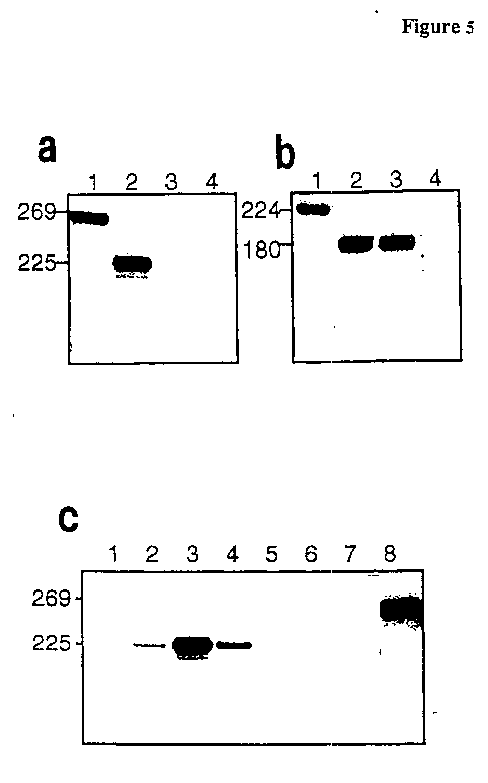

### Figure 5. RNAse protection analysis of CIITA expression.

10 µg of total RNA were hybridized in 80% formamide to 500,000 cpm of probe. Protected probe was resolved on denaturing 6% polyacrylamide gels.
(a, b) Total RNA of Raji (lane 2) and RJ2.25 (lane 3) was hybridized to riboprobes complementary to nucleotides 2049 to 1824 (a) and nucleotides 4520 to 4340 (b) of CIITA respectively. Undigested probed and yeast RNA control are shown in lanes 1 and 4.
(c) Total RNA from various cell lines was hybridized to the same central CIITA probe used in (a). 1, yeast control; 2, CO115 colon carcinoma; 3, 4, Mann, QBL B-LCLs; 5, 2102Ep teratocarcinoma; 6, MOLT4 T-lymphoma; 7, SK-N-AS neuroblastoma; 8, undigested probe. RNA quantity and quality was controlled by hybridization to a TBP specific probe (not shown).

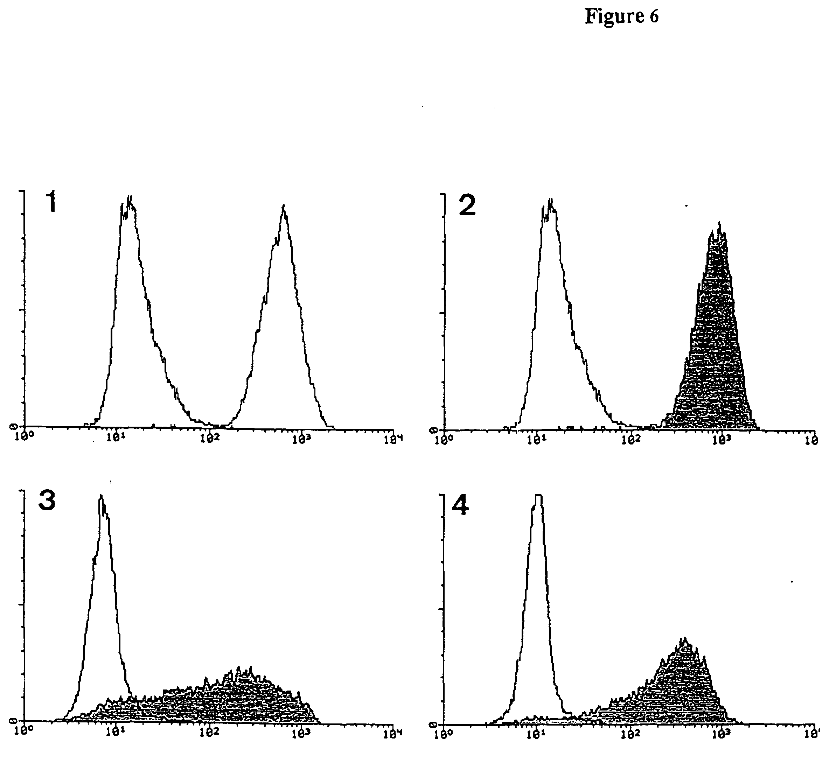

### Figure 6. CIITA cDNA corrects three different HLA class II negative mutant cell lines.

RJ2.25 (panel 2), RM3 (panel 3) or REM-34 (panel 4) cells were transfected with CIITA cDNA, cloned into EBO-Sfi (shaded profiles) or with EBO-Sfi alone (open profiles). Analysis for surface HLA DR expression by FACS with L243 antibody was performed after two weeks (RJ2.25, RM3) or only five days (REM-34) of Hygromycin B selection. Panel 1 shows negative (RJ2.25; EBO-Sfi transfected) and positive (Raji) controls.

Finally, this invention is illustrated by the examples which follow. This section is included in order to aid in an understanding of the invention but is not intended to, and should not be construed to, limit the invention as et forth in the claims in any way.

### Figure 7. CIITA cDNA fully corrects HLA class II expression on B lymphocytes from HLA class II deficient patients.

B lymphocytes from patient BLS1 were transfected with CIITA cDNA. Following selection in Hygromycin, cells were analyzed for surface expression of HLA-DR, -DP and -DQ.

### Figure 8. The CIITA gene induces expression of MHC class II genes in class II negative cells.

Three types of MHC class II negative cells were transfected with CIITA cDNA. Following selection in Hygromycin, cells were analyzed for surface expression of HLA-DR molecules.

### A. A melanoma cell line. B. A fibroblastic cell line, 143B. C. HeLa cells

### Figure 9. Activation of human T cell clones with CIITA transfectants and interferon-stimulated cells used as APC's.

Three types of cells were tested as APC'S: Untransfected melanoma cell line, MHC class II negative (•); The same cell line following 48 hours of exposure to 200 µg/ml of interferon gamma, MHC class II positive (■); The same cell line previously transfected with CIITA cDNA, MHC class II positive (▲). These cells were treated with various concentrations of p4, a tetanus toxoid peptide, at the concentrations indicated, fixed by UV light and incubated with two different human T cell clones specific for that peptide, T-50 and T-87. The extent of T cell activation was measured by incorporation of H³-thymidine in a standard assay, and expressed as "stimulation index".

### Example 1

### Expression cloning by complementation of mutant RJ2.25

Expression cloning by genetic complementation of the class II regulatory mutant RJ2.25 implies no bias as to the nature of the genetic defect but requires that the defect concern a single gene. In addition to using re-expression of the endogenous HLA class II genes as a selection marker, the inventors constructed a series of cDNA expression vectors that allowed several different selection strategies, either separately or in combination.

### Construction of the cDNA expression vector EBO-Sfi

The cDNA expression vector EBO-Sfi (Figure 1) was constructed in the following way. The Hygromycin B resistance-conferring gene hph of EBO-pLPP [Spickofsky et al., DNA Prot. Engin. Techn. 2 (1990), 14-18 (gift of Dr. R.F. Margolskee)] was replaced by the optimized hph gene of pTG76 [Giordano et al., loc. cit. (gift of Dr. W.T. McAllister)]. The hph-76 gene under the control of the SV40 early promoter was isolated from pTG76 as a 2,546 bp fragment resulting from partial PstI and complete BamHI digestion (positions 1845 and 4391 in pTG76). From EBO-pLPP a 6,675 bp PstI-EcoRV (positions 1859, 5765) and a 615 bp BamHI-EcoRV fragment (positions 5141, 5756) were isolated and ligated with the hph-76 PstI-BamHI fragment. In the resulting vector the SfiI sites in both SV40 early promoters were destroyed by SfiI-digestion, 3' overhang removal and re-ligation; this did not affect the promoter activity. Next, the EBO-pLPP polylinker (SacI to KpnI, positions 1 to 37 in EBO-pLPP) was replaced by a SfiI-cDNA cloning cassette. For this an 800 bp bacterial chloramphenicol acetyl transferase (CAT) gene was inserted into the SacI and KpnI sites with the help of oligonucleotide adaptors. In the resulting EBO-Sfi vector the sequences constituting the cDNA cloning cassette are as follows: 5' GAGCTCGGCCTCACTGGCC-CAT-gene-GGCCAGTGAGGCCGGTACC 3' (SEQ ID NO: 1, SEQ ID NO: 2).

### Construction of the vectors DV and DRA-CD

Vector DV (Figure 1C) was generated by replacing the hph-76 gene controlling SV40 early promoter between the unique HindIII and BgIII sites in EBO-Sfi with a 300 bp fragment of the HLA DRA promoter (position -270 to position +30; relative to the transcription start site).

In vector DV, the Hygromycin B resistance gene is placed under the control of the HLA-DRA promoter. This allows antibiotic selection for reactivation of HLA class II transcription.

For the construction of DRA-CD (Figure 1B) a reporter gene cassette consisting of the 300 bp HLA DRA promoter, the 1,742 bp human CD4 cDNA gene [Maddon et al., loc. cit.] and the 134 bp SmaI-BamHI poly-adenylation signal of pTG76 was inserted into the HindIII site of EBO-Sfi. For cDNA cloning, plasmid DNA was digested to completion with SfiI and separated from the CAT-stuffer on a 5%-20% sucrose gradient.

### Construction of cDNA library and plasmid pools

Double-stranded cDNA was synthesized from 20 µg of polyadenylated mRNA prepared from Raji cells with Superscript reverse transcriptase (Gibco-BRL). The cDNA was ligated to non-palindromic SfiI-SalI adaptors (5'pTGGCCGTCGACTAC [SEQ ID NO: 3], 5'pGTAGTCGACGGCCAGTG [SEQ ID NO: 4]). Adaptor-ligated cDNA was size-fractioned on a 5%-20% sucrose gradient and inserts greater than 2.5 kb were ligated into the plasmid vectors DV and DRA-CD, respectively. The ligation reactions were electroporated into Escherichia coli strain DH5A, yielding a potential library titer of more than 5 x 10⁷ recombinants for this size fraction. The mean insert size of cDNAs after SalI or SfiI digestion was approximately 3-3.5 kb. Recombinants were plated out at 5 x 10⁴ colonies per 15 cm petri dish with Luria-broth agar containing 50 µg/ml ampicillin. Cells were scraped off the plates in pools of 5 x 10⁵ recombinants and were partially stored as glycerol stocks at -70°C, the remaining bacteria being used to isolate plasmid DNA by the alkaline lysis miniprep method [Sambrook et al., Molecular Cloning, A Laboratory Manual, Cold Spring Harbor Laboratory Press (1989)].

### RJ2.25 transfections

Cells were grown in an RPMI 1640 medium supplemented with 10% fetal calf serum, penicillin, streptomycin, and glutamine at 37°C in 5% CO₂.

RJ2.25 were recloned by limiting dilution before the transfection experiments. Fifty million RJ2.25 cells were transfected by electroporation with 20 ul of plasmid DNA and 400 ug E. coli tRNA as carrier in 800 ul RPMI medium. One ml of FCS was added to each electroporation cuvette immediately after delivery of the electric pulse. Forty-eight hours after transfection, Hygromycin B (Calbiochem) was added at 125 ug/ml; the dose was increased by two steps in three day intervals to a final concentration of 250 ug/ml. Cell numbers were kept between 1 x 10⁵ and 1 x 10⁶/ml. Hygromycin B selection was complete after two weeks. Transfection efficiencies as determined by limiting dilution experiments were routinely greater than 25% stable transfectants with EBO-Sfi and DRA-CD plasmids. DV-plasmids yielded 10%-15% stable transfectants when transfected into Raji cells; after transfection into RJ2.25 greater, 99.9% of the cells died under Hygromycin B selection.

### Plow cytometric analyses

The following HLA class II specific monoclonal antibodies were used: 2.06 (DR, gift of C. Epplen), L243 (DR, ), B7/21 (DP, gift of N. Reinsmoen), Tü22 (DQ, gift of A. Ziegler). In addition, we used CD4 specific OKT4 monoclonal antibody (gift of M. Hadam).

Cells (10⁶) were incubated in staining buffer (phosphate-buffered saline, 1% bovine serum albumin and 2.5% heat-inactivated human AB serum) with monoclonal antibodies for 30 min at 4°C. The cells were washed and incubated with FITC-labeled rabbit anti-mouse IgG (SEROTEC). After washing, 10,000 viable cells were analyzed using a FACScan flow cytometer (Becton-Dickinson). Dead cells were excluded from the analysis by staining them with propidium iodide and by their forward and sideways light scattering properties.

### Cell sorting

The transfected RJ2.25 cells were subjected to a first round of sorting three to five days after transfection. Between 3 x 10⁷ and 1.2 x 10⁸ cells were used per transfection and sorting step. Cells were collected by centrifugation, washed once with PBS/BSA and then stained with 2.06 or OKT4 ascites antibody in PBS/BSA in the presence of 2.5% heat-inactivated human AB-serum for 30 min. on ice. After washing, the cells were incubated with 5-10 ul goat-anti-mouse coupled magnetic beads (Dynal, Norway) in PBS/BSA supplemented with 2.5% heat-inactivated goat serum for 30 min. on ice. Bead-coupled cells were separated from unstained cells with the help of a magnet, washed several times and expanded. The beads stick for several days to the cells but do not inhibit growth and were not removed. Further rounds of selection were carried out when the cell populations had grown up to sufficient numbers (3-5 x 10⁷). Hygromycin B selection was maintained during this whole process, although the concentration of the antibiotic was sometimes reduced for one or two days after bead selection to optimize cell survival. The sorted populations were analyzed for HLA DR expression by staining them with 2.06 or L243 and FACS analysis after each round of selection. After two rounds of immuno-selection in addition to Hygromycin B selection, the cultures of transfectants obtained with three out of five DV-cDNA pools exhibited strong HLA-DR expression (Figure 2A, panels 1, 2, 3).

### Plasmid rescue

Plasmids were isolated from sorted cell populations by alkaline lysis in close analogy to bacterial miniprep methods. Rescued plasmid pools were transfected by electroporation into E. coli strain DHlOB (generous gift of Gibco-BRL). Several hundred colonies were scraped from agar plates, expanded, and plasmid DNA was extracted. Inserts of these plasmid pools were analyzed by digestion with SfiI, Sall, SacI and KpnI followed by gel electrophoresis. Plasmid DNA from randomly picked single colonies was analyzed in the same way and plasmids containing the same insert type as the one predominantly found in the rescued pools were used for retransfection into RJ2.25 cells.

Plasmid pools rescued from these highly selected populations exhibited a limited set of characteristic insert-derived bands following digestion with SalI, SfiI, SacI and KpnI and gel electrophoresis. The same 4.5 kb cDNA insert was found in over 50% of randomly picked bacterial colonies obtained with the rescued plasmids. The transfectants selected for Hygromycin B resistance only were negative when tested for HLA-DR expression. Plasmid pools rescued from these cells showed a random distribution of insert sizes.

Finally, RJ2.25 mutant cells stably transfected with cDNA libraries prepared in vector DRA-CD were sorted by immuno-selection, both for expression of HLA-DR and for CD4 expression (with 2.06 and OKT4 antibodies and magnetic beads). After three rounds of sorting, the selected transfected populations showed clear HLA-DR or CD4 expression, albeit somewhat weaker than the RJ2.25/DV pools described above (Figure 2A, panel 4). About 30% of the plasmids rescued from the cells sorted for HLA-DR expression alone showed the same preferential 4.5 kb insert as the one found in the DV transfectants (see above). In contrast, plasmids rescued from transfectants selected for CD4 expression alone also displayed a preferential but different cDNA insert, which upon retransfection in mutant cells had no effect on HLA class II expression (not shown). These plasmids have not yet been analyzed further.

### Example 2

### CIITA cDNA restores expression of a full wild type HLA class II phenotype in the mutant cell line.

Plasmids from individual colonies containing the 4.5 kb cDNA insert found preferentially in selected transfectants re-expressing HLA-DR (see above) were isolated and then re-transfected into class II negative mutant RJ2.25 B cells. Transfectants were then selected for Hygromycin B resistance only. Without any immuno-selection for HLA class II expression, stable transfectants expressed wild type levels of HLA-DR (Figure 2B, panel 2). Identical results were obtained in these complementation experiments whether the Hygromycin resistance gene was driven by an SV40 promoter or by a DRA promoter. The cDNA found capable of restoring expression of HLA-DR genes in the regulatory mutant cell line was called CIITA, for "Class II transactivator."

Expression of the different HLA class II genes is generally regulated in a global fashion [Mach et al., Cold Spring Harb. Symp. Quant. Biol. 51 (1986), 67-74], although exceptions have been described. Cell surface analysis of RJ2.25 mutant cells stably transfected with CIITA cDNA showed not only expression of wild type levels of HLA-DR molecules, the class II molecules used for the initial selection of corrected cells, but also demonstrated re-expression of the HLA-DP and HLA-DQ isotypes (Figure 2B, panel 2-4). The CIITA transactivator affected in RJ2.25 is therefore sufficient to correct for the expression of all HLA-DR, -DQ and -DP a and b chain genes and to restore in the mutant cell line a normal and complete HLA class II positive phenotype.

### Example 3

### Sequence of CIITA cDNA and Protein

Double-stranded DNA was sequenced by the dideoxy chain termination method using mostly the T7 G/A-deaza sequencing kit of Pharmacia but also the Bst sequencing kit of Biorad. The cDNA and translated protein sequence were tested for homology to sequences in the EMBL (release 33), GenBank (release 74), SWISS-PROT (release 24), and dbEST databases. Sequence analysis was performed with PC/Gene release 6.7 (Intelligenetics), the BLAST server, and the PROSITE dictionary.

The nucleotide and deduced amino acid sequence of CIITA cDNA are presented in Figure 3. The 5' ends of CIITA cDNA clones obtained from several independent sorting experiments were sequenced and their starting points are indicated in the figure. Clone pDVP10-1 was sequenced completely. It is 4,543 bp in length and contains an open reading frame of 3390 bp, coding for a predicted protein of 1130 amino acids, with a start codon at nucleotide position 116. There is a stop codon in the same reading frame at position 20-22. Although this first in-frame ATG codon (position 116) fulfills the most important criteria for a translation initiation site, a second in-frame ATG codon, at bp 188 (a.a. position 25), is in the context of a perfect "Kozak box" and may also serve as a start-codon, leading to a protein of 1106 amino acids. The 1130 amino acid protein has a predicted molecular weight of 123.5 kD, which corresponds well to an apparent molecular weight of ¼ 135-140 kD observed for the protein translated in vitro from CIITA cDNA (not shown). The 5' UT region is 115 bp long and the 3' UT region is 1 kb long.

Apart from Alu-repeats in the 3' untranslated region of the CIITA cDNA (bp 3890 to 4190), no significant homologies to either nucleotide or protein sequences in available data bases were detected. A search for known or potential functional motives within the predicted CIITA protein sequence revealed a potential ATP/GTP binding site at aa position 420 to 427 (Figure 3). The N-terminal portion of the protein shows a region rich in acidic residues (aa 30 to 160, see Figure 3), with the sequence spanning residues 50 to 137 containing 30% glutamate/aspartate. Residues 162 to 322 are rich in proline, serine and threonine. Within this region, three stretches (residues 163-195, 209-237 and 260-322) show 20% to 23% proline and 17% to 28% serine/threonine. CIITA is therefore a novel gene whose function is essential to MHC class II gene expression in B lymphocytes.

### Example 4

### Identification of the CIITA-gene defect in the mutant B cell line

Since the HLA class II negative mutant RJ2.25 was generated by low dose irradiation, which is known to induce deletions and chromosome rearrangements, we analyzed the organization of the CIITA gene in RJ2.25 by Southern blot hybridization.

High molecular weight DNA was extracted from the cultured cells [Miller et al., Nucl. Acids Res. 16 (1988), 1215]. Restriction endonuclease-digested DNA was separated on 0.7% agarose/TBE gels and capillary-transferred onto positively charged nylon membranes (Boehringer-Mannheim). Southern hybridization was carried out with random-nonamer ³²P labeled cDNA fragments in Quick-Hybridization Mix (Stratagene) according to the manufacturer's instructions in the presence of sonicated single-stranded human placenta DNA as competitor. The Blots were washed twice in 2 x SSC, 0.1% SDS at 40°C, followed by two washes in 0.1 x SSC, 0.1% SDS at 65°C before exposure to X-AR film with intensifying screens at -70°C.

HindIII digested genomic DNA from the parental B cell line Raji showed three positive bands after hybridization with a full length CIITA cDNA probe, as well as a high background signal due to the Alu-repeat sequence described in the 3' untranslated region of the CIITA cDNA (see above). Hybridization was therefore performed with distinct fragments of the CIITA cDNA. As shown in Figure 4B, the 1.8 kb central HindIII fragment of CIITA cDNA hybridizes to a 3.8 kb HindIII fragment in genomic DNA of the parental B cell line Raji. This band is completely absent in DNA from the mutant cell line RJ2.25 (Figure 4B, probe b), even after prolonged exposure of the blots, indicating deletion of that central region of the CIITA gene on both chromosomes. The genomic HindIII fragments that hybridize respectively to a 5' and a 3' CIITA cDNA probe are both present, unaltered in length, in DNA from the mutant cell line (Figure 4B, probes a and c). These two bands, however, display only half the signal intensity in RJ2.25 compared to wild type Raji DNA, suggesting the presence of only one copy of each of these two fragments in the mutant cell.

Finally, the analysis of CIITA mRNA by RNAse protection showed, as expected from the complete deletion documented by Southern blots, an absence of signal with a probe located within the deleted central part of the CIITA cDNA (Figure 5). Interestingly, a probe corresponding to the 3' end of CIITA mRNA was protected (Figure 5B), suggesting readthrough across the internal deletion observed in the mutated gene. These experiments offer direct evidence for the absence of an intact CIITA gene in regulatory mutant RJ2.25 and indicate that the irradiation used for the generation of the mutant line had lead to the complete loss of one CIITA allele, while inducing an internal deletion in the other.

### Example 5

### Other HLA class II regulatory mutants are also corrected by CIITA.

HLA class II negative regulatory mutant B lymphoblastoid cell lines have been generated independently of RJ2.25, and by different procedures, including different protocols of mutagenesis. The inventors tested two additional such cell lines to explore whether, like in RJ2.25 (Figure 6, panel 2), CIITA cDNA could correct for HLA-DR expression. Mutant RM3 [Calman et al., J. Immunol. 139 (1987), 2489-2495] was produced by chemical mutagenesis and shown to correspond, like RJ2.25, to a recessive defect in HLA class II regulation. When class II negative RM3 mutant B cells were transfected with CIITA cDNA, normal expression of HLA-DR was restored, as shown in Figure 6, panel 3. As in the case of the RJ2.25 mutant, HLA-DQ and -DP expression were restored as well (data not shown). The inventors also generated HLA class II-negative mutants from the same Raji parental B cell line, following mutagenesis with EMS. One such mutant (REM-34) was transfected with CIITA cDNA and analyzed for HLA-DR expression. As in the two previous cases, CIITA had restored expression of HLA class II molecules (Figure 6, panel 4).

### Differential expression of CIITA

MHC class II genes are only expressed in a limited number of cell types. To date however, the factors suspected of being involved in the control of MHC class II expression have not exhibited a pattern of expression that correlates with that of MHC class II genes [Glimcher et al., loc. cit.]. In order to address this question for CIITA, RNAse protection experiments were performed with several MHC class II negative and positive cell lines (Fig. 5c).

Two CIITA cDNA fragments were prepared as RNAse protection probes. The internal probe covers nucleotides 2049 (SfiI) to 1824 (NcoI) protecting 225 bp of CIITA mRNA. The 3' end probe is complementary to a 180 bp fragment spanning nucleotides 4520 to 4340 (Hinfl) of CIITA. As a control a fragment was prepared which protects a 275 bp fragment of (bp 1228 to 953) of the TBP mRNA. From the linearized constructs ³²P-UTP labeled riboprobes were generated by in-vitro transcription leaving 44, 44, and 60 bp non-complementary vector encoded overhangs. Hybridization of 10 ug of total RNA completed to 50 ug with yeast RNA was carried out in 80% formamide with 500,000 cpm of probe over night at 50°C.

After digestion with RNAses A and T1 [Sambrook et al., loc. cit.], the protected fragments were resolved by electrophoresis on 6% polyacrylamide, 8 M urea gels and visualized by autoradiography. Several EBV transformed B cell lines, as well as the class II positive colon carcinoma cell line CO115, showed expression of CIITA mRNA. In contrast, three HLA class II negative cell lines, 2102Ep, Andrews MOLT-4 and SK-N-AS, showed no signal for CIITA mRNA expression (Figure 5C). From this initial study, the inventors concluded that CIITA expression is not ubiquitous but is itself regulated, with a pattern that seems to correlate with MHC class II expression.

### Example 6

### CIITA cDNA can restore the expression of MHC class II molecules in cells from patients with hereditary MHC class II deficiency.

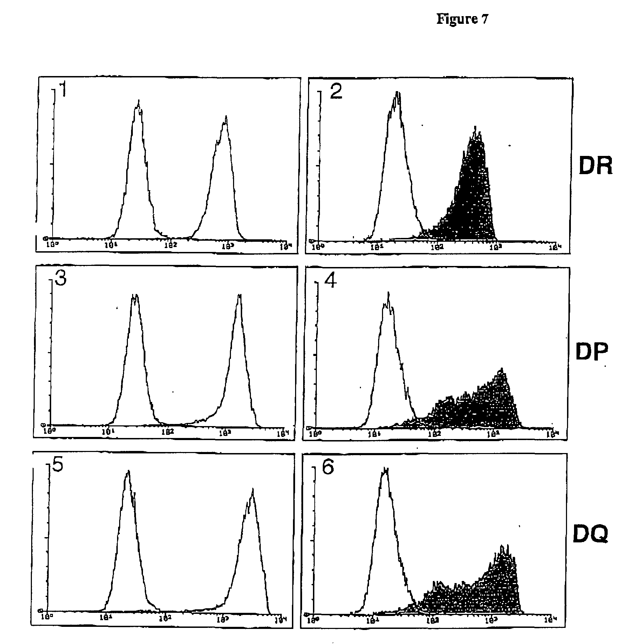

Hereditary MHC class II deficiency is a form of primary immunodeficiency with a total lack of expression of MHC class II genes. Patients suffering from this disease frequently die from multiple infections. The CIITA gene was found to correct the genetic defect in some of these patients (see Figure 7). B lymphocytes from patients with MHC class II deficiency can be transfected with the CIITA cDNA in an appropriate vector which results in the re-expression of all MHC class II genes. This correction of a genetic defect in live cells by a cloned gene opens the way to gene therapy for this disease.

### Example 7

### CIITA cDNA can induce the expression of MHC class molecules in different types of MHC class II negative cells.

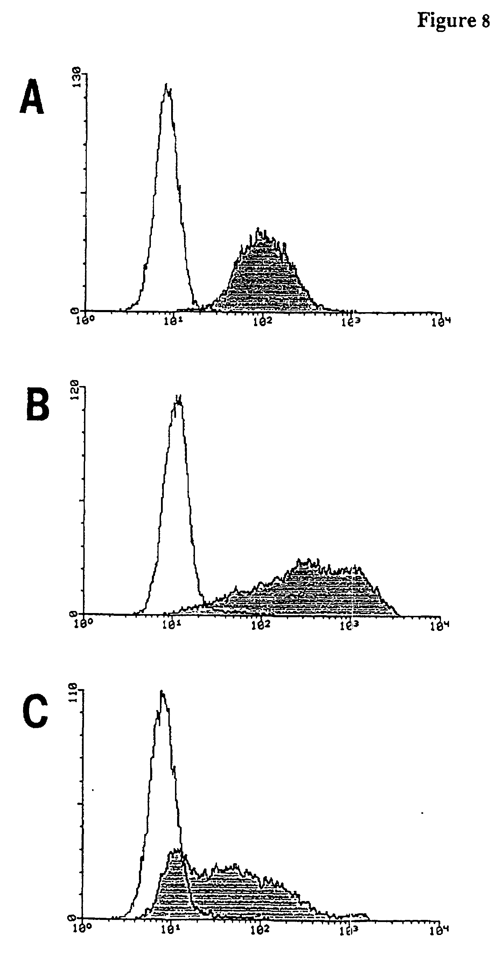

Most of the cells of the body are normally MHC class II negative. In a number of situations, such as vaccination or induction of an immune response against cancer cells, it is desirable to induce or boost the expression of MHC class II genes in cells that are normally class II negative, in order to render these cells more immunogenic. The data illustrated in Figure 8 show that this can indeed be achieved. Three different MHC class II negative human cell lines were transfected with CIITA cDNA, which was sufficient to induce expression of high levels of MHC class II surface molecules. This effect of CIITA cDNA has obvious implications for new strategies for vaccination and for gene therapy in cancer.

### Example 8

### MHC class II molecules induced by the transactivator gene CIITA are highly efficient in peptide binding and in peptide-specific T lymphocyte activation.

Cells where the expression of MHC class II molecules had been induced by the transactivator gene CIITA (as CIITA cDNA) were tested for their ability to bind exogenously provided peptides and to present such peptides to T cell clones. These CIITA transfectants were compared with the same cells where MHC class II expression had been induced by interferon gamma. The two types of cells expressed MHC class II molecules at the surface at a similar level. This experiment was done with T cell clones of the appropriate HLA-DR specificity and exhibiting specificity for the particular tetanus toxoid peptides used. Peptide-specific T cell activation by these two types of MHC class II positive cells was measured by H³-thymidine incorporation, expressed as a stimulation index. In this particular experiment, melanoma cells were used as antigen-presenting cells.

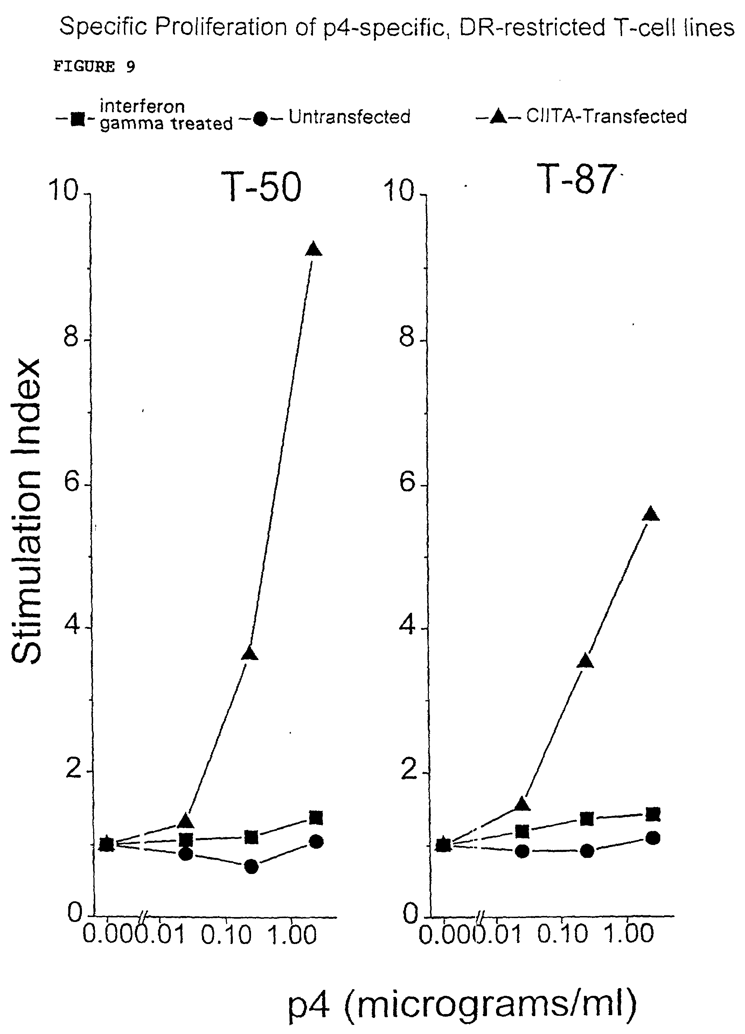

As can be seen in Figure 9, MHC class II negative, untransfected control cells failed to activate T lymphocytes. Interferon gamma stimulated, MHC class II positive cells also failed to induce significant T cell activation, even at high peptide concentration. On the other hand, cells transfected with CIITA cDNA behave as highly efficiently antigen presenting cells and peptide-specific T cell activators. In agreement with these functional results, the peptide binding ability of these two types of MHC class II positive cells was drastically different, being low in the interferon activated cells and high in the CIITA transfectants. Thus, CIITA induces the expression of MHC class II molecules that are unusual in their highly efficient capacity to bind and to present peptides to T lymphocytes, leading to activation of these lymphocytes. CIITA can therefore be used to generate cells that exhibit a very high peptide-specific immunogenicity.

## Claims

1. A gene coding for a protein displaying MHC class II transactivator (CIITA) activity, said protein being essential for the general control of MHC class II gene expression, said gene being selected from:
(a) the DNA sequence shown in Figure 3 (SEQ ID NO:5);
(b) DNA sequences which hybridize to the DNA sequence of (a) under stringent hybridization conditions;
(c) allelic derivatives or fragments of the DNA sequence of (a) or (b); or
(d) DNA sequences which are degenerate as a result of the genetic code to the DNA sequence of (a), (b), or (c).

2. The DNA sequence of claim 1(b) which is a genomic DNA sequence.

3. The gene of claim 1, wherein said CIITA has an amino acid sequence as shown in Figure 3 (SEQ ID NO:6)

4. Antisense RNA or DNA sequences **characterized in that** they are complementary to the genes of any one of claims 1 to 3, said sequences being capable of inhibiting the synthesis of the proteins encoded by said genes.

5. Ribozymes **characterized in that** they are complementary to the genes of any one of claims 1 to 3 and can selectively bind to and cleave said genes, thus inhibiting the synthesis of the proteins encoded by said genes.

6. A recombinant vector comprising the gene of any one of claims 1 to 5.

7. The recombinant vector of claim 6 in which the inserted gene is in operable linkage with an expression control sequence.

8. The recombinant vector according to claim 7 which is pDVP10-1 (DSM 8422).

9. A host cell being transformed with a recombinant vector according to any one of claims 6 to 8.

10. A method for the identification of MHC class II transactivators, said method comprising
(a) construction of a cDNA expression library from the mRNA of Raji cells using a vector containing an MHC class II promoter driving a gene for antibiotic resistance;
(b) transforming an MHC class II mutant cell line with the DNA of said library;
(c) screening the transformants by genetic complementation using antibiotic selection for reactivation of MHC class II transcription and re-expressing of the endogenous MHC class II gene as selection markers; and
(d) isolating and sequencing plasmids which show antibiotic resistance and complementation.

11. A protein encoded by a gene of any one of claims 1 to 3 displaying CIITA activity and being essential for the general control of MHC class II gene expression in vertebrate cells.

12. The protein according to claim 11, wherein the vertebrate cells are mammalian, preferably human cells.

13. The protein according to claim 11 or 12 which is a recombinant protein.

14. Use of the protein of claim 13 for the identification of inhibitors to proteins displaying CIITA activity.

15. A method for the production of a protein according to claim 13 comprising cultivating a host cell of claim 9 under conditions allowing the expression of said protein and recovering said protein from the culture.

16. A pharmaceutical composition containing the substance according to any one of claims 1 to 8 or 11 to 13.

17. The pharmaceutical composition of claim 16 containing proteins according to any one of claims 11 to 13 or a nucleic acid according to any one of claims 1 to 3 or 6 to 8 for the treatment of diseases associated with impaired expression of MHC class II genes where an increase in MHC class II gene expression is desirable.

18. The pharmaceutical composition of claim 16 containing proteins according to any one of claims 11 to 13 or a nucleic acid according to any one of claims 1 to 3 or 6 to 8 for use in vaccination and in gene therapy for cancer, where an increase in MHC class II expression is desirable.

19. The pharmaceutical composition of claim 16 containing a nucleic acid according to any one of claims 1 to 3 or 6 to 8 for boosting peptide-specific immunogenicity of various cell types.

20. The pharmaceutical composition of claim 16 containing a nucleic acid according to claim 4 or 5 for the treatment of diseases associated with an aberrant expression of MHC class II genes where a decrease of the level of the expression of MHC class II genes is desirable or for the generation of MHC class II negative transgenic animals as a source of organs for xenogenic transplantation or of cells for universal cell transplants.

21. A process for the isolation of a CIITA inhibitor, comprising screening for molecules capable of binding to the protein of any one of claims 11 to 13 and selecting those molecules that inhibit the expression of MHC class II genes.

22. A process for the isolation of a CIITA inhibitor, comprising designing structural analogous of the protein of any one of claims 11 and 13 and selecting those structural analogous acting as antagonists.

## Patentansprüche

1. Gen, welches ein Protein mit MHC-Klasse II-Transaktivatoraktivität (CIITA) codiert, wobei dieses Protein notwendig für die generelle Kontrolle der MHC-Klasse II-Genexpression ist und das Gen ausgewählt ist aus:
(a) der DNA-Sequenz, wie sie in Figur 3 dargestellt ist (SEQ ID NO: 5);
(b) DNA-Sequenzen, die mit der DNA-Sequenz aus (a) unter stringenten Hybridisierungsbedingungen hybridisieren;
(c) allelischen Derivaten oder Fragmenten der DNA-Sequenz aus (a) oder (b); oder
(d) DNA-Sequenzen, die aufgrund des genetischen Codes degeneriert sind zu einer DNA-Sequenz aus (a), (b) oder (c).

2. DNA-Sequenz nach Anspruch 1(b), die eine genomische DNA-Sequenz ist.

3. Gen nach Anspruch 1, wobei CIITA eine Aminosäuresequenz besitzt, wie sie in Figur 3 dargestellt ist (SEQ ID NO: 6).

4. Antisense-RNA- oder Antisense-DNA-Sequenzen, **dadurch gekennzeichnet, dass** die komplementär zu Genen nach einem der Ansprüche 1 bis 3 sind, wobei die Sequenzen geeignet sind zur Hemmung der Synthese der Proteine, für die diese Gene codieren.

5. Ribozyme, **dadurch gekennzeichnet, dass** sie komplementär zu Genen nach einem der Ansprüche 1 bis 3 sind und selektiv an die Gene binden und diese spalten können, wobei dadurch die Synthese der Proteine, die von diesen Genen codiert werden, gehemmt wird.

6. Rekombinanter Vektor, umfassend das Gen nach einem der Ansprüche 1 bis 5.

7. Rekombinanter Vektor nach Anspruch 6, in dem das inserierte Gen funktionell mit einer Expressionskontrollsequenz verbunden ist.

8. Rekombinanter Vektor nach Anspruch 7, der pDVP10-1 (DSM 8422) ist.

9. Wirtszelle, die mit einem rekombinanten Vektor nach einem der Ansprüche 6 bis 8 transformiert ist.

10. Verfahren zur Identifizierung von MHC-Klasse II-Transaktivatoren, umfassend:
(a) Konstruktion einer cDNA-Expressionsbibliothek aus der mRNA von Raji-Zellen unter Verwendung eines Vektors umfassend einen MHC-Klasse II-Promotor, der ein Gen für eine Antibiotikaresistenz steuert;
(b) Transformieren einer MHC-Klasse ll-Mutanten-Zelllinie mit der DNA dieser Bibliothek;
(c) Durchmustern der Transformanten durch genetische Komplementation unter Verwendung einer antibiotischen Selektion zur Reaktivierung der MHC-Klasse II-Transkription und Reexpression des endogenen MHC-Klasse II-Gens als Selektionsmarkern; und
(d) Isolieren und Sequenzieren von Plasmiden, die Antibiotikaresistenz und Komplementation zeigen.

11. Protein, codiert von einem Gen nach einem der Ansprüche 1 bis 3, welches CIITA-Aktivität besitzt und notwendig für die generelle Kontrolle der MHC-Klasse II-Genexpression in Vertebraten-Zellen ist.

12. Protein nach Anspruch 11, wobei die Vertebraten-Zellen Säugerzellen, vorzugsweise menschliche Zellen sind.

13. Protein nach Anspruch 11 oder 12, welches ein rekombinantes Protein ist.

14. Verwendung des Proteins nach Anspruch 13 zur Identifizierung von Inhibitoren für Proteine, welche CIITA-Aktivität besitzen.

15. Verfahren zur Herstellung eines Proteins nach Anspruch 13, umfassend das Züchten einer Wirtszelle nach Anspruch 9 unter Bedingungen, die die Expression des Proteins erlauben, und Gewinnen des Proteins aus der Kultur.

16. Arzneimittel, enthaltend den Stoff nach einem der Ansprüche 1 bis 8 oder 11 bis 13.

17. Arzneimittel nach Anspruch 16, enthaltend Proteine nach einem der Ansprüche 11 bis 13 oder eine Nucleinsäure nach einem der Ansprüche 1 bis 3 oder 6 bis 8 zur Behandlung von Erkrankungen, die mit einer Störung der Expression von MHC-Klasse II-Genen in Verbindung stehen, wobei eine Steigerung der MHC-Klasse II-Genexpression angestrebt ist.

18. Arzneimittel nach Anspruch 16, enthaltend Proteine nach einem der Ansprüche 11 bis 13 oder eine Nucleinsäure nach einem der Ansprüche 1 bis 3 oder 6 bis 8 zur Verwendung bei der Impfung und Gentherapie für Krebs, wobei eine Steigerung der MHC-Klasse II-Expression angestrebt ist.

19. Arzneimittel nach Anspruch 16, enthaltend eine Nucleinsäure nach einem der Ansprüche 1 bis 3 oder 6 bis 8 zur Steigerung der peptidspezifischen Immunogenität von verschiedenen Zelltypen.

20. Arzneimittel nach Anspruch 16, enthaltend eine Nucleinsäure nach Anspruch 4 oder 5, zur Behandlung von Krankheiten, die mit einer abnormen Expression von MHC-Klasse II-Genen in Verbindung stehen, wobei eine Abnahme der Höhe der Expression von MHC-Klasse II-Genen angestrebt ist, oder zur Erzeugung von MHC-Klasse II-negativen transgenen Tieren als eine Quelle für Organe für xenogene Transplantation oder von Zellen für universelle Zelltransplantate.

21. Verfahren zur Isolierung eines CIITA-Inhibitors, umfassend die Durchmusterung auf Moleküle mit der Fähigkeit zur Bindung an das Protein nach einem der Ansprüche 11 bis 13 und das Auswählen solcher Moleküle, welche die Expression von MHC-Klasse II-Genen hemmen.

22. Verfahren zur Isolierung eines CIITA-Inhibitors, umfassend das Entwerfen von Strukturanalogen des Proteins nach einem der Ansprüche 11 und 13 und das Auswählen solcher Strukturanalogen, die als Antagonisten wirken.

## Revendications

1. Gène codant pour une protéine possédant une activité de transactivateur (CIITA) de la classe II de MHC, ladite protéine étant essentielle au contrôle général de l'expression du gène de la classe II de MHC, ledit gène étant choisi parmi :
(a) la séquence d'ADN montrée sur la Figure 3 (SEQUENCE ID N°:5) ;
(b) les séquences d'ADN qui s'hybrident à la séquence d'ADN de (a) dans des conditions d'hybridation stringentes
(c) les dérivés ou fragments alléliques de la séquence d'ADN de (a) ou (b) ; ou
(d) les séquences d'ADN qui sont dégénérées par l'influence du code génétique sur les séquences d'ADN (a), (b) ou (c)

2. Séquence d'ADN selon la revendication 1(b) qui est une séquence d'ADN génomique.

3. Gène selon la revendication 1, dans laquelle ledit CIITA renferme une séquence d'acides aminés telle que montrée sur la Figure 3 (SEQUENCE ID N°:6).

4. Séquences d'ADN ou d'ARN antisens **caractérisées en ce qu'**elles sont complémentaires du gène selon l'une quelconque des revendications 1 à 3,lesdites séquences étant capable d'inhiber la synthèse des protéines codées par lesdits gènes.

5. Ribozymes **caractérisées en ce qu'**elles sont complémentaires des gènes selon l'une quelconque des revendications 1 à 3 et peuvent se fixer sélectivement auxdits gènes et les cliver, inhibant ainsi la synthèse des protéines codées par lesdits gènes.

6. Vecteur recombinant renfermant le gène selon l'une quelconque des revendications 1 à 5.

7. Vecteur recombinant selon la revendication 6, dans lequel le gène inséré est en liaison activable avec une séquence de contrôle de l'expression.

8. Vecteur recombinant selon la revendication 7 qui est pDVP10-1 (DSM 8422).

9. Cellule hôte qui est transformée avec un vecteur recombinant selon l'une quelconque des revendications 6 à 8.

10. Procédé d'identification de transactivateurs de la classe II de MHC, ledit procédé comprenant les étapes consistant à :
(a) procéder à la construction d'une bibliothèque d'expressions d'ADNc à partir de l'ARNm de cellules de Raji en utilisant un vecteur, renfermant un promoteur de la classe II de MHC, porteur d'un gène conférant une résistance à un antibiotique ;
(b) transformer une souche cellulaire mutante de la classe II de MHC avec l'ADN de ladite bibliothèque ;
(c) trier les transformants par complémentation génétique en utilisant la sélection par antibiotique pour la réactivation de la transcription de la classe II de MHC et la ré-expression du gène de la classe II de MHC endogène comme marqueurs de sélection ; et
(d) isoler et séquencer les plasmides qui présentent la résistance à l'antibiotique et la complémentation.

11. Protéine codée par un gène selon la revendication 1 à 3 possédant une activité de CIITA et étant essentielle au contrôle général de l'expression du gène de la classe II de MHC dans les cellules de vertébrés.

12. Protéine selon la revendication 11, dans laquelle les cellules de vertébrés sont des cellules de mammifères, de préférence, des cellules humaines.

13. Protéine selon la revendication 11 ou 12 qui est une protéine recombinante.

14. Utilisation de la protéine recombinante selon la revendication 13 pour l'identification d'inhibiteurs de protéines possédant l'activité de CIITA.

15. Procédé de production d'une protéine selon la revendication 13 comprenant la culture d'une cellule hôte selon la revendication 9 dans des conditions autorisant l'expression de ladite protéine et la récupération de ladite protéine à partir de la culture.

16. Composition pharmaceutique renfermant la substance selon l'une quelconque des revendications précédentes 1 à 8 ou 11 à 13.

17. Composition pharmaceutique renfermant la substance de la revendication 16 selon l'une quelconque des revendications 11 à 13 ou un acide nucléique selon l'une quelconque des revendications précédentes 1 à 3 ou 6 à 8 pour le traitement de maladies associées à l'expression défectueuse de gènes de la classe II de MHC où une augmentation de l'expression de la classe II est désirable.

18. Composition pharmaceutique selon la revendication 16 renfermant des protéines selon l'une quelconque des revendications précédentes 11 à 13 ou un acide nucléique selon l'une quelconque des revendications précédentes 1 à 3 ou 6 à 8 pour utilisation dans la vaccination et dans la thérapie génique du cancer où une augmentation de l'expression de la classe II de MHC est désirable.

19. Composition pharmaceutique renfermant un acide nucléique selon l'une quelconque des revendications précédentes 1 à 3 ou 6 à 8 pour amplifier l'immunogénicité de divers types de cellules spécifiquement conférée par un peptide.

20. Composition pharmaceutique selon la revendication 16 renfermant un acide nucléique selon la revendication 4 ou 5 pour le traitement de maladies associées à une expression aberrante de gènes de la classe II de MHC où une diminution du niveau d'expression de gènes de la classe II de MHC est désirable ou pour la génération d'animaux transgéniques négatifs de la classe II de MHC comme sources d'organes destinés à une transplantation xénogène ou de cellules destinées à former des transplants cellulaires universels.

21. Procédé d'isolement d'un inhibiteur du CIITA comprenant le criblage de molécules capables de se lier à la protéine selon l'une quelconque des revendications 11 à 13 et la sélection des molécules qui inhibent l'expression de gènes de la classe II de MHC.

22. Procédé d'isolement d'un inhibiteur du CIITA comprenant la construction par ingénierie génétique d'analogues structuraux de la protéine selon l'une quelconque des revendications 11 et 13 et la sélection des analogues structuraux agissant comme antagonistes.


### SEQUENCE LISTING

(1) GENERAL INFORMATION:
   (i) APPLICANT:
      (A) NAME: MACH, Bernard
      (B) STREET: 45, route de Pregny
      (C) CITY: Chambésy
      (E) COUNTRY: Switzerland
      (F) POSTAL CODE (ZIP): 1292
   (ii) TITLE OF INVENTION: Proteins Essential for the Expression of Vertebrate MHC Class II Genes, DNA Sequences Encoding Same and Pharmaceutical Compositions
   (iii) NUMBER OF SEQUENCES: 6
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS :
      (A) LENGTH: 19 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iii) ANTI-SENSE: NO
   (xi) SEQUENCE DESCRIPTION: SEQ ID NO: 1:
   GAGCTCGGCC TCACTGGCC                                              19
(2) INFORMATION FOR SEQ ID NO: 2:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iii) ANTI-SENSE: NO
   (xi) SEQUENCE DESCRIPTION: SEQ ID NO: 2:
   GGCCAGTGAG GCCGGTACC 19
(2) INFORMATION FOR SEQ ID NO: 3:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: CDNA
   (iii) HYPOTHETICAL: NO
   (iii) ANTI-SENSE: NO
   (xi) SEQUENCE DESCRIPTION: SEQ ID NO: 3:
   TGGCCGTCGA CTAC 14
(2) INFORMATION FOR SEQ ID NO: 4:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iii) ANTI-SENSE: NO
   (xi) SEQUENCE DESCRIPTION: SEQ ID NO: 4:
   GTAGTCGACG GCCAGTG 17
(2) INFORMATION FOR SEQ ID NO: 5:
   (i) SEQUENCE CHARACTERISTICS:
      (A) LENGTH: 4543 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 5:
   TGATGAGGCT GTGTGCTTCT GAGCTGGGCA TCCGAAGGCA TCCTTGGGGA AGCTGAGGGC     60
   ACGAGGAGGG GCTGCCAGAC TCCGGGAGCT GCTGCCTGGC TGGGATTCCT ACACAATGCG    120
   TTGCCTGGCT CCACGCCCTG CTGGGTCCTA CCTGTCAGAG CCCCAAGGCA GCTCACAGTG    180
   TGCCACCATG GAGTTGGGGC CCCTAGAAGG TGGCTACCTG GAGCTTCTTA ACAGCGATGC    240
   TGACCCCCTG TGCCTCTACC ACTTCTATGA CCAGATGGAC CTGGCTGGAG AAGAAGAGAT    300
   TGAGCTCTAC TCAGAACCCG ACACAGACAC CATCAACTGC GACCAGTTCA GCAGGCTGTT    360
   GTGTGACATG GAAGGTGATG AAGAGACCAG GGAGGCTTAT GCCAATATCG CGGAACTGGA    420
   CCAGTATGTC TTCCAGGACT CCCAGCTGGA GGGCCTGAGC AAGGACATTT TCAAGCACAT    480
   AGGACCAGAT GAAGTGATCG GTGAGAGTAT GGAGATGCCA GCAGAAGTTG GGCAGAAAAG    540
   TCAGAAAAGA CCCTTCCCAG AGGAGCTTCC GGCAGACCTG AAGCACTGGA AGCCAGCTGA    600
   GCCCCCCACT GTGGTGACTG GCAGTCTCCT AGTGGGACCA GTGAGCGACT GCTCCACCCT    660
   GCCCTGCCTG CCACTGCCTG CGCTGTTCAA CCAGGAGCCA GCCTCCGGCC AGATGCGCCT    720
   GGAGAAAACC GACCAGATTC CCATGCCTTT CTCCAGTTCC TCGTTGAGCT GCCTGAATCT    780
   CCCTGAGGGA CCCATCCAGT TTGTCCCCAC CATCTCCACT CTGCCCCATG GGCTCTGGCA    840
   AATCTCTGAG GCTGGAACAG GGGTCTCCAG TATATTCATC TACCATGGTG AGGTGCCCCA    900
   GGCCAGCCAA GTACCCCCTC CCAGTGGATT CACTGTCCAC GGCCTCCCAA CATCTCCAGA    960
   CCGGCCAGGC TCCACCAGCC CCTTCGCTCC ATCAGCCACT GACCTGCCCA GCATGCCTGA   1020
   ACCTGCCCTG ACCTCCCGAG CAAACATGAC AGAGCACAAG ACGTCCCCCA CCCAATGCCC   1080
   GGCAGCTGGA GAGGTCTCCA ACAAGCTTCC AAAATGGCCT GAGCCGGTGG AGCAGTTCTA   1140
   CCGCTCACTG CAGGACACGT ATGGTGCCGA GCCCGCAGGC CCGGATGGCA TCCTAGTGGA   1200
   GGTGGATCTG GTGCAGGCCA GGCTGGAGAG GAGCAGCAGC AAGAGCCTGG AGCGGGAACT   1260
   GGCCACCCCG GACTGGGCAG AACGGCAGCT GGCCCAAGGA GGCCTGGCTG AGGTGCTGTT   1320
   GGCTGCCAAG GAGCACCGGC GGCCGCGTGA GACACGAGTG ATTGCTGTGC TGGGCAAAGC   1380
   TGGTCAGGGC AAGAGCTATT GGGCTGGGGC AGTGAGCCGG GCCTGGGCTT GTGGCCGGCT   1440
   TCCCCAGTAC GACTTTGTCT TCTCTGTCCC CTGCCATTGC TTGAACCGTC CGGGGGATGC   1500
   CTATGGCCTG CAGGATCTGC TCTTCTCCCT GGGCCCACAG CCACTCGTGG CGGCCGATGA   1560
   GGTTTTCAGC CACATCTTGA AGAGACCTGA CCGCGTTCTG CTCATCCTAG ACGCCTTCGA   1620
   GGAGCTGGAA GCGCAAGATG GCTTCCTGCA CAGCACGTGC GGACCGGCAC CGGCGGAGCC 1680
   CTGCTCCCTC CGGGGGCTGC TGGCCGGCCT TTTCCAGAAG AAGCTGCTCC GAGGTTGCAC 1740
   CCTCCTCCTC ACAGCCCGGC CCCGGGGCCG CCTGGTCCAG AGCCTGAGCA AGGCCGACGC 1800
   CCTATTTGAG CTGTCCGGCT TCTCCATGGA GCAGGCCCAG GCATACGTGA TGCGCTACTT 1860
   TGAGAGCTCA GGGATGACAG AGCACCAAGA CAGAGCCCTG ACGCTCCTCC GGGACCGGCC 1920
   ACTTCTTCTC AGTCACAGCC ACAGCCCTAC TTTGTGCCGG GCAGTGTGCC AGCTCTCAGA 1980
   GGCCCTGCTG GAGCTTGGGG AGGACGCCAA GCTGCCCTCC ACGCTCACGG GACTCTATGT 2040
   CGGCCTGCTG GGCCGTGCAG CCCTCGACAG CCCCCCCGGG GCCCTGGCAG AGCTGGCCAA 2100
   GCTGGCCTGG GAGCTGGGCC GCAGACATCA AAGTACCCTA CAGGAGGACC AGTTCCCATC 2160
   CGCAGACGTG AGGACCTGGG CGATGGCCAA AGGCTTAGTC CAACACCCAC CGCGGGCCGC 2220
   AGAGTCCGAG CTGGCCTTCC CCAGCTTCCT CCTGCAATGC TTCCTGGGGG CCCTGTGGCT 2280
   GGCTCTGAGT GGCGAAATCA AGGACAAGGA GCTCCCGCAG TACCTAGCAT TGACCCCAAG 2340
   GAAGAAGAGG CCCTATGACA ACTGGCTGGA GGGCGTGCCA CGCTTTCTGG CTGGGCTGAT 2400
   CTTCCAGCCT CCCGCCCGCT GCCTGGGAGC CCTACTCGGG CCATCGGCGG CTGCCTCGGT 2460
   GGACAGGAAG CAGAAGGTGC TTGCGAGGTA CCTGAAGCGG CTGCAGCCGG GGACACTGCG 2520
   GGCGCGGCAG CTGCTTGAGC TGCTGCACTG CGCCCACGAG GCCGAGGAGG CTGGAATTTG 2580
   GCAGCACGTG GTACAGGAGC TCCCCGGCCG CCTCTCTTTT CTGGGCACCC GCCTCACGCC 2640
   TCCTGATGCA CATGTACTGG GCAAGGCCTT GGAGGCGGCG GGCCAAGACT TCTCCCTGGA 2700
   CCTCCGCAGC ACTGGCATTT GCCCCTCTGG ATTGGGGAGC CTCGTGGGAC TCAGCTGTGT 2760
   CACCCGTTTC AGGGCTGCCT TGAGCGACAC GGTGGCGCTG TGGGAGTCCC TGCGGCAGCA 2820
   TGGGGAGACC AAGCTACTTC AGGCAGCAGA GGAGAAGTTC ACCATCGAGC CTTTCAAAGC 2880
   CAAGTCCCTG AAGGATGTGG AAGACCTGGG AAAGCTTGTG CAGACTCAGA GGACGAGAAG 2940
   TTCCTCGGAA GACACAGCTG GGGAGCTCCC TGCTGTTCGG GACCTAAAGA AACTGGAGTT 3000
   TGCGCTGGGC CCTGTCTCAG GCCCCCAGGC TTTCCCCAAA CTGGTGCGGA TCCTCACGGC 3060
   CTTTTCCTCC CTGCAGCATC TGGACCTGGA TGCGCTGAGT GAGAACAAGA TCGGGGACGA 3120
   GGGTGTCTCG CAGCTCTCAG CCACCTTCCC CCAGCTGAAG TCCTTGGAAA CCCTCAATCT 3180
   GTCCCAGAAC AACATCACTG ACCTGGGTGC CTACAAACTC GCCGAGGCCC TGCCTTCGCT 3240
   CGCTGCATCC CTGCTCAGGC TAAGCTTGTA CAATAACTGC ATCTGCGACG TGGGAGCCGA 3300
   GAGCTTGGCT CGTGTGCTTC CGGACATGGT GTCCCTCCGG GTGATGGACG TCCAGTACAA 3360
   CAAGTTCACG GCTGCCGGGG CCCAGCAGCT CGCTGCCAGC CTTCGGAGGT GTCCTCATGT 3420
   GGAGACGCTG GCGATGTGGA CGCCCACCAT CCCATTCAGT GTCCAGGAAC ACCTGCAACA 3480
   ACAGGATTCA CGGATCAGCC TGAGATGATC CCAGCTGTGC TCTGGACAGG CATGTTCTCT 3540
   GAGGACACTA ACCACGCTGG ACCTTGAACT GGGTACTTGT GGACACAGCT CTTCTCCAGG 3600
   CTGTATCCCA TGAGGCCTCA GCATCCTGGC ACCCGGCCCC TGCTGGTTCA GGGTTGGCCC 3660
   CTGCCCGGCT GCGGAATGAA CCACATCTTG CTCTGCTGAC AGACACAGGC CCGGCTCCAG 3720
   GCTCCTTTAG CGCCCAGTTG GGTGGATGCC TGGTGGCAGC TGCGGTCCAC CCAGGAGCCC 3780
   CGAGGCCTTC TCTGAAGGAC ATTGCGGACA GCCACGGCCA GGCCAGAGGG AGTGACAGAG 3840
   GCAGCCCCAT TCTGCCTGCC CAGGCCCCTG CCACCCTGGG GAGAAAGTAC TTCTTTTTTT 3900
   TTATTTTTAG ACAGAGTCTC ACTGTTGCCC AGGCTGGCGT GCAGTGGTGC GATCTGGGTT 3960
   CACTGCAACC TCCGCCTCTT GGGTTCAAGC GATTCTTCTG CTTCAGCCTC CCGAGTAGCT 4020
   GGGACTACAG GCACCCACCA TCATGTCTGG CTAATTTTTC ATTTTTAGTA GAGACAGGGT 4080
   TTTGCCATGT TGGCCAGGCT GGTCTCAAAC TCTTGACCTC AGGTGATCCA CCCACCTCAG 4140
   CCTCCCAAAG TGCTGGGGAT TACAAGCGTG AGCCACTGCA CCGGGCCACA GAGAAAGTAC 4200
   TTCTCCACCC TGCTCTCCGA CCAGACACCT TGACAGGGCA CACCGGGCAC TCAGAAGACA 4260
   CTGATGGGCA ACCCCCAGCC TGCTAATTCC CCAGATTGCA ACAGGCTGGG CTTCAGTGGC 4320
   AGGCTGCTTT TGTCTATGGG ACTCAATGCA CTGACATTGT TGGCCAAAGC CAAAGCTAGG 4380
   CCTGGCCAGA TGCACCAGGC CCTTAGCAGG GAAACAGCTA ATGGGACACT AATGGGGCGG 4440
   TGAGAGGGGA ACAGACTGGA AGCACAGCTT CATTTCCTGT GTCTTTTTTC ACTACATTAT 4500
   AAATGTCTCT TTAATGTCAC AAAAAAAAAA AAAAAAAAAA AAA 4543
(2) INFORMATION FOR SEQ ID NO: 6:
   (i) SEQUENCE CHARACTERISTICS:
      (A) LENGTH: 1130 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 6:
   Met Arg Cys Leu Ala Pro Arg Pro Ala Gly Ser Tyr Leu Ser Glu Pro
   1 5 10 15
   Gln Gly Ser Ser Gln Cys Ala Thr Met Glu Leu Gly Pro Leu Glu Gly
               20                  25                  30
   Gly Tyr Leu Glu Leu Leu Asn Ser Asp Ala Asp Pro Leu Cys Leu Tyr
           35                  40                  45
   His Phe Tyr Asp Gln Met Asp Leu Ala Gly Glu Glu Glu Ile Glu Leu
       50                  55                  60
   Tyr Ser Glu Pro Asp Thr Asp Thr Ile Asn Cys Asp Gln Phe Ser Arg
   65                  70                  75                  80
   Leu Leu Cys Asp Met Glu Gly Asp Glu Glu Thr Arg Glu Ala Tyr Ala
                   85                  90                  95
   Asn Ile Ala Glu Leu Asp Gln Tyr Val Phe Gln Asp Ser Gln Leu Glu
               100                 105                 110
   Gly Leu Ser Lys Asp Ile Phe Lys His Ile Gly Pro Asp Glu Val Ile
           115                 120                 125
   Gly Glu Ser Met Glu Met Pro Ala Glu Val Gly Gln Lys Ser Gln Lys
       130                 135                 140
   Arg Pro Phe Pro Glu Glu Leu Pro Ala Asp Leu Lys His Trp Lys Pro
   145                 150                 155                 160
   Ala Glu Pro Pro Thr Val Val Thr Gly Ser Leu Leu Val Gly Pro Val
                   165                 170                 175
   Ser Asp Cys Ser Thr Leu Pro Cys Leu Pro Leu Pro Ala Leu Phe Asn
               180                 185                 190
   Gln Glu Pro Ala Ser Gly Gln Met Arg Leu Glu Lys Thr Asp Gln Ile
           195                 200                 205
   Pro Met Pro Phe Ser Ser Ser Ser Leu Ser Cys Leu Asn Leu Pro Glu
       210                 215                 220
   Gly Pro Ile Gln Phe Val Pro Thr Ile Ser Thr Leu Pro His Gly Leu
   225                 230                 235                 240
   Trp Gln Ile Ser Glu Ala Gly Thr Gly Val Ser Ser Ile Phe Ile Tyr
                   245                 250                 255
   His Gly Glu Val Pro Gln Ala Ser Gln Val Pro Pro Pro Ser Gly Phe
               260                 265                 270
   Thr Val His Gly Leu Pro Thr Ser Pro Asp Arg Pro Gly Ser Thr Ser
           275                 280                 285
   Pro Phe Ala Pro Ser Ala Thr Asp Leu Pro Ser Met Pro Glu Pro Ala
       290                 295                 300
   Leu Thr Ser Arg Ala Asn Met Thr Glu His Lys Thr Ser Pro Thr Gln
   305                 310                 315                 320
   Cys Pro Ala Ala Gly Glu Val Ser Asn Lys Leu Pro Lys Trp Pro Glu
                   325                 330                 335
   Pro Val Glu Gln Phe Tyr Arg Ser Leu Gln Asp Thr Tyr Gly Ala Glu
               340                 345                 350
   Pro Ala Gly Pro Asp Gly Ile Leu Val Glu Val Asp Leu Val Gln Ala
           355                 360                 365
   Arg Leu Glu Arg Ser Ser Ser Lys Ser Leu Glu Arg Glu Leu Ala Thr
       370                 375                 380
   Pro Asp Trp Ala Glu Arg Gln Leu Ala Gln Gly Gly Leu Ala Glu Val
   385                 390                 395                 400
   Leu Leu Ala Ala Lys Glu His Arg Arg Pro Arg Glu Thr Arg Val Ile
                   405                 410                 415
   Ala Val Leu Gly Lys Ala Gly Gln Gly Lys Ser Tyr Trp Ala Gly Ala
               420                 425                 430
   Val Ser Arg Ala Trp Ala Cys Gly Arg Leu Pro Gln Tyr Asp Phe Val
           435                 440                 445
   Phe Ser Val Pro Cys His Cys Leu Asn Arg Pro Gly Asp Ala Tyr Gly
       450                 455                 460
   Leu Gln Asp Leu Leu Phe Ser Leu Gly Pro Gln Pro Leu Val Ala Ala
   465                 470                 475                 480
   Asp Glu Val Phe Ser His Ile Leu Lys Arg Pro Asp Arg Val Leu Leu
                   485                 490                 495
   Ile Leu Asp Ala Phe Glu Glu Leu Glu Ala Gln Asp Gly Phe Leu His
               500                 505                 510
   Ser Thr Cys Gly Pro Ala Pro Ala Glu Pro Cys Ser Leu Arg Gly Leu
           515                 520                 525
   Leu Ala Gly Leu Phe Gln Lys Lys Leu Leu Arg Gly Cys Thr Leu Leu
       530                 535                 540
   Leu Thr Ala Arg Pro Arg Gly Arg Leu Val Gln Ser Leu Ser Lys Ala
   545                 550                 555                 560
   Asp Ala Leu Phe Glu Leu Ser Gly Phe Ser Met Glu Gln Ala Gln Ala
                   565                 570                 575
   Tyr Val Met Arg Tyr Phe Glu Ser Ser Gly Met Thr Glu His Gln Asp
               580                 585                 590
   Arg Ala Leu Thr Leu Leu Arg Asp Arg Pro Leu Leu Leu Ser His Ser
           595                 600                 605
   His Ser Pro Thr Leu Cys Arg Ala Val Cys Gln Leu Ser Glu Ala Leu
       610                 615                 620
   Leu Glu Leu Gly Glu Asp Ala Lys Leu Pro Ser Thr Leu Thr Gly Leu
   625                 630                 635                 640
   Tyr Val Gly Leu Leu Gly Arg Ala Ala Leu Asp Ser Pro Pro Gly Ala
                   645                 650                 655
   Leu Ala Glu Leu Ala Lys Leu Ala Trp Glu Leu Gly Arg Arg His Gln
               660                 665                 670
   Ser Thr Leu Gln Glu Asp Gln Phe Pro Ser Ala Asp Val Arg Thr Trp
           675                 680                 685
   Ala Met Ala Lys Gly Leu Val Gln His Pro Pro Arg Ala Ala Glu Ser
       690                 695                 700
   Glu Leu Ala Phe Pro Ser Phe Leu Leu Gln Cys Phe Leu Gly Ala Leu
   705                 710                 715                 720
   Trp Leu Ala Leu Ser Gly Glu Ile Lys Asp Lys Glu Leu Pro Gln Tyr
                   725                 730                 735
   Leu Ala Leu Thr Pro Arg Lys Lys Arg Pro Tyr Asp Asn Trp Leu Glu
               740                 745                 750
   Gly Val Pro Arg Phe Leu Ala Gly Leu Ile Phe Gln Pro Pro Ala Arg
           755                 760                 765
   Cys Leu Gly Ala Leu Leu Gly Pro Ser Ala Ala Ala Ser Val Asp Arg
       770                 775                 780
   Lys Gln Lys Val Leu Ala Arg Tyr Leu Lys Arg Leu Gln Pro Gly Thr
   785                 790                 795                 800
   Leu Arg Ala Arg Gln Leu Leu Glu Leu Leu His Cys Ala His Glu Ala
                   805                 810                 815
   Glu Glu Ala Gly Ile Trp Gln His Val Val Gln Glu Leu Pro Gly Arg
               820                 825                 830
   Leu Ser Phe Leu Gly Thr Arg Leu Thr Pro Pro Asp Ala His Val Leu
           835                 840                 845
   Gly Lys Ala Leu Glu Ala Ala Gly Gln Asp Phe Ser Leu Asp Leu Arg
       850                 855                 860
   Ser Thr Gly Ile Cys Pro Ser Gly Leu Gly Ser Leu Val Gly Leu Ser
   865                 870                 875                 880
   Cys Val Thr Arg Phe Arg Ala Ala Leu Ser Asp Thr Val Ala Leu Trp
                   885                 890                 895
   Glu Ser Leu Arg Gln His Gly Glu Thr Lys Leu Leu Gln Ala Ala Glu
               900                 905                 910
   Glu Lys Phe Thr Ile Glu Pro Phe Lys Ala Lys Ser Leu Lys Asp Val
           915                 920                 925
   Glu Asp Leu Gly Lys Leu Val Gln Thr Gln Arg Thr Arg Ser Ser Ser
       930                 935                 940
   Glu Asp Thr Ala Gly Glu Leu Pro Ala Val Arg Asp Leu Lys Lys Leu
   945                 950                 955                 960
   Glu Phe Ala Leu Gly Pro Val Ser Gly Pro Gln Ala Phe Pro Lys Leu
                   965                 970                 975
   Val Arg Ile Leu Thr Ala Phe Ser Ser Leu Gln His Leu Asp Leu Asp
               980                 985                 990
   Ala Leu Ser Glu Asn Lys Ile Gly Asp Glu Gly Val Ser Gln Leu Ser
           995                 1000                1005
   Ala Thr Phe Pro Gln Leu Lys Ser Leu Glu Thr Leu Asn Leu Ser Gln
       1010                1015                1020
   Asn Asn Ile Thr Asp Leu Gly Ala Tyr Lys Leu Ala Glu Ala Leu Pro
   1025                1030                1035                1040
   Ser Leu Ala Ala Ser Leu Leu Arg Leu Ser Leu Tyr Asn Asn Cys Ile
                   1045                1050                1055
   Cys Asp Val Gly Ala Glu Ser Leu Ala Arg Val Leu Pro Asp Met Val
               1060                1065                1070
   Ser Leu Arg Val Met Asp Val Gln Tyr Asn Lys Phe Thr Ala Ala Gly
           1075                1080                1085
   Ala Gln Gln Leu Ala Ala Ser Leu Arg Arg Cys Pro His Val Glu Thr
       1090                1095                1100
   Leu Ala Met Trp Thr Pro Thr Ile Pro Phe Ser Val Gln Glu His Leu
   1105                1110                1115                1120
   Gln Gln Gln Asp Ser Arg Ile Ser Leu Arg
                   1125                1130